# EUROPEAN PATENT APPLICATION

(11) **EP 4 299 082 A1**
(43) Date of publication of application: **03.01.2024**
(21) Application number: 21927085.7
(22) Date of filing: 26.02.2021
(51) Int. Cl.: A61L 9/14, A61L 9/12, A41G 1/00, B60H 3/00, A61L 9/04

(54) **FRAGRANCE-RELEASE DEVICE**

(71) Applicant: Noar Brasil Indústria e Comércio de Fragrâncias Digitais Ltda, 04535-110 São Paulo - SP (BR)
(72) Inventor: GALVÃO, Cláudia, 05440-000 São Paulo - SP (BR); FERREIRA, Tulio Silvio, 35660-105 Pará de Minas - MG (BR)
(74) Representative: Torner, Juncosa I Associats, SL
(86) International application number: PCT/BR2021/050082
(87) International publication number: WO 2022/178597

(57) **Abstract**

The present invention relates to a device comprising a base (2) containing the refill (5 or 6) and a couplable lid (3) in which an air pump (12) actuated locally or remotely via a messaging application, forces the air of the housing (13) towards an ambient-air intake duct (8) pressurizing the container (4) with the refill (5 or 6), which naturally directs the air towards the fragranced-air outlet duct (9), releasing a dry jet of air through the fragranced-air overflow channel (20), located in the lid (3) of the device (1).

## Description

### INTRODUCTION

The present patent application is related to an unprecedented fragrance-release device, basically constituted of a base, with a container holding the essence, adaptable to a lid with the operational components which convert the essence into a spray of dry air released into the ambience by an electronic communication command via message apps.

### FIELD OF INVENTION

The invention in question is applied in the entertainment, electronic communication and perfumery segments.

### CONVINCING

People have been routinely communicating by means of electronic messages for some time, both in writing as by audios and videos exchanged by apps that are available in the network, which with the faster internet connection bands were widely disseminated.

In this context, new technologies are developed based on digital platforms seeking to rescue from the virtual world sensations which provoke the senses of human being, such as, for example, the olfactory memory.

### STATE OF THE ART

The current state of the art anticipates some patent documents which teach about toys and ornaments that release aroma, such as **PI 0405675-2** entitled "AUTOMATIC REGENERATIVE AROMATIC SYSTEM" - can be installed in ornaments, knickknacks, vases, stuffed toys, among other ornaments. It is constituted by a sensor or timer which has the purpose of activating the liquid lift pump motor; interconnecting wire between the sensor or timer with the liquid lift pump; liquid lift pump; spray tube with the purpose of conducting and spraying the aromatic liquid; aromatic liquid container; siphon tube with the purpose of preventing the aromatic liquid contained in the aromatic liquid container from losing its aroma and "optional" fan which has the purpose of releasing the aroma forcedly.

The system of the above document presents a series of technical limitations, beginning by the inability of releasing a dry spray of air from the aroma, since it uses a liquid essence pumping system with optional ventilation, thus compromising the real scent of the aroma. Another negative point is that due to the components and the constructive arrangement thereof, it is not possible to use same in small size ornaments. Using liquid essence where the possibility of leaking is high is also inconvenient. Finally, the activation is not performed remotely, but by means of the timer.

Document US 2006/0222560 entitled "STUFFED TOY AIR-FRAGRANCE DISPENSING DEVICE" -fragrance source disposed within a container contained within the stuffed toy. It is actuated by a battery located within the toy, which activates a fan and a heat source, which in turn will move the air past the fragrance source through the hollow stem being dispensed by an upper opening.

The stuffed toy of the above document presents a fragrance dispensing device which uses heat to dispense the fragrance, which is not safe for this type of children's product. Additionally, the components used do not enable the adequation thereof to any type of ornament and toy other than the stuffed one. The emission of the scent or aroma by heating and heat source directly on the essence, apart from degenerating the real aroma, causes high energy consumption. Whereby the emission of aromas by thermal means significantly alters the olfactory notes and the accuracy of the scent emitted.

Document US8,408,481 entitled *"MOBILE FRAGRANCE-DISPENSING DEVICE RESEMBLING A PET'* - presents a mobile fragrance-dispensing device resembling a pet. A dispenser system functions to spray fragrance from said fragrance canister when actuated by microprocessor.

The device presented above uses fragrance flasks which are found in the market which are actuated by a motor, according to a schedule made by a microprocessor. In the above solution it is not possible to use a specific container or refill customized according to the final ornament of the application in question.

Document US 2015/290548 Entitled " *TOY MESSAGING SYSTEM'* - refers to a stuffed doll comprising a body; a highspeaker; an antenna for receiving a wireless data transmission from a mobile device; storage means to store said data transmission; a processor for processing said data transmission; and a media device for reproducing media files.

The above cited doll receives data; however, it does not interact with message apps.

### OBJECT OF THE INVENTION

It is an object of the present invention to provide a device for releasing fragrances in ornaments constituted by two parts which are adaptable to each other, being a base containing a container with the essence of a certain fragrance and another part a lid with the operational components that transform and exhale said essence in an air spray, duly maintaining the scent thereof;

It is an object of the present invention to propose a device for releasing fragrances in ornaments which due to the fact that it is comprised by two parts can assume the appearance of a compact three-dimensional object, such as a toy, a keychain, or any other ornament;

It is an object of the present invention to propose a device for releasing fragrances in ornaments which essences can be more fluid, contained in absorbent refills or else more viscous, deposited at the bottom of the container itself;

It is an object of the present invention to propose a device for releasing fragrances in ornaments which does not use heating for dispensing the fragrance in the form of a dry air spray, maintaining the olfactory notes and the accuracy of the emitted scent;

It is an object of the present invention to propose a device for releasing fragrances in ornaments having a reduced number of operational components and complements, being therefore of great constructive simplicity regardless of the outer contour of the ornament;

It is an object of the present invention to propose a device for releasing fragrances in ornaments actuated locally or remotely by means of electronic text, video or audio message, thus approximating the virtual world to the real experience by awakening the olfactory memory of the user;

It is an object of the present invention to propose a device for releasing fragrances in ornaments having a great cost-benefit ratio.

### SUMMARY OF THE INVENTION

The invention is related to a device with reduced dimensions formed by a base and a lid which are adaptable to each other and which functions locally or by means of electronic message that is configurable according to the wish of the user. The base comprises a capsule which holds a container containing the refill with the essence of a determined aroma, as well as a pump suction tube with two ducts, whereby one is an ambient-air intake duct and the other the scented air outlet duct. The lid holds the feeding and energizing part of the circuit and the air pump forces the flow toward the interior of the device through a side air intake and directs it to the container by the air intake duct, which container when pressurized exhales the dry air spray through the outlet air duct and from there to the diffusion channel which has the outlet point on the lid of the device. The base and the lid, due to the constructive simplicity and reduced number of components, can assume different three-dimensional forms, in this embodiment of the invention the shape of a miniature doll.

When the device is energized and has its battery charged it goes into operation automatically. In the remote mode by means of internet or data connection, the device is paired with a message app and the users can send their aroma at a certain moment, adjusting the intensity and the duration of the emission. After sending the trigger command by the sender, and acceptance by the receiver, the electronic system is actuated causing the emission of scented air to the receiver of the message.

### DESCRIPTION OF THE FIGURES

The invention will now be described in the best embodiment thereof, whereby, for a better understanding, reference will be made to the attached drawings, wherein there are represented:

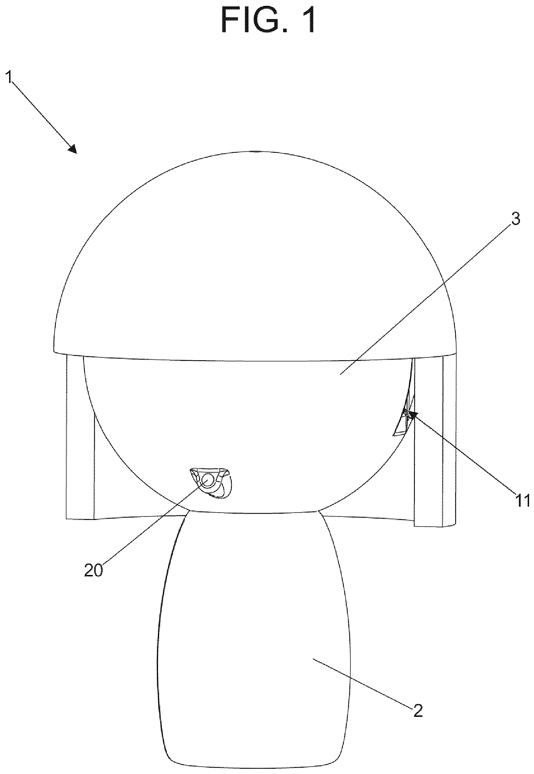

**FIGURE 1****:** Perspective view of the fragrance-release devices;

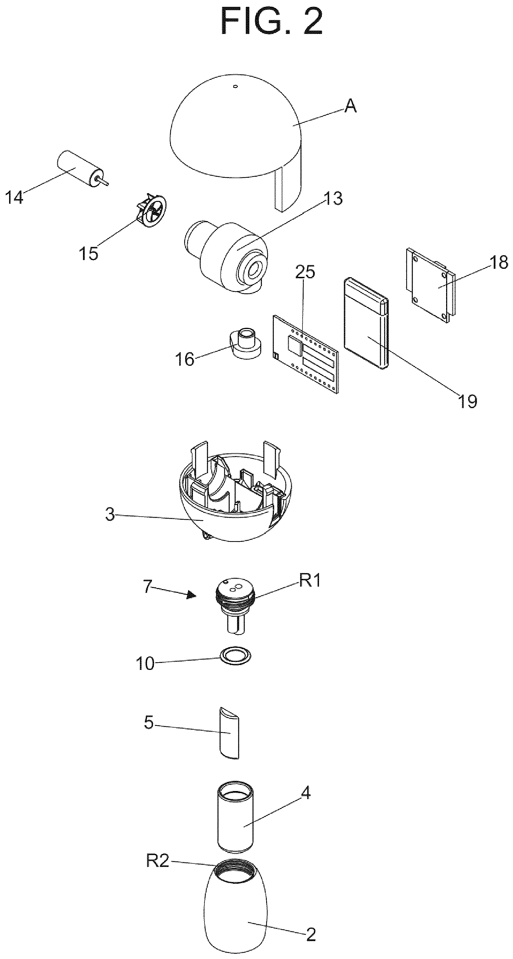

**FIGURE 2****:** Exploded perspective view of the fragrance-release devices;

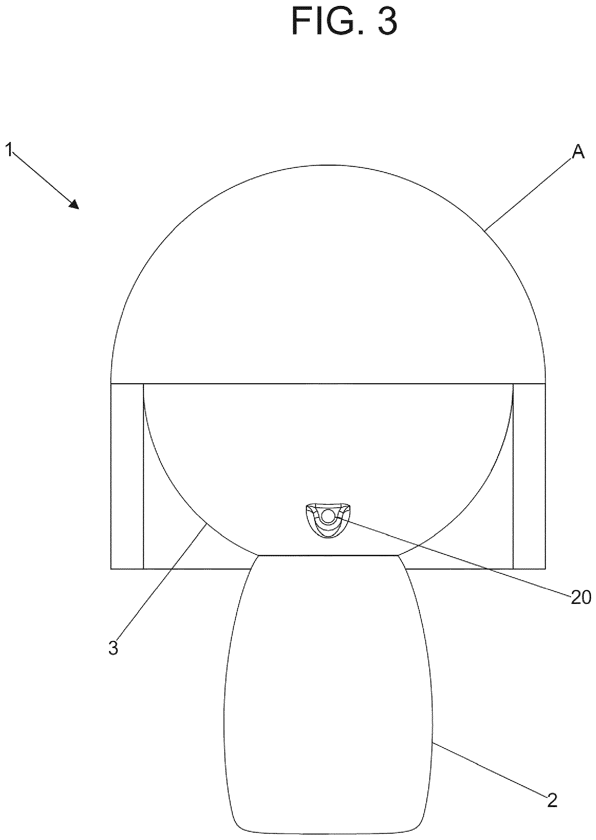

**FIGURE 3****:** Front view of the fragrance-release devices;

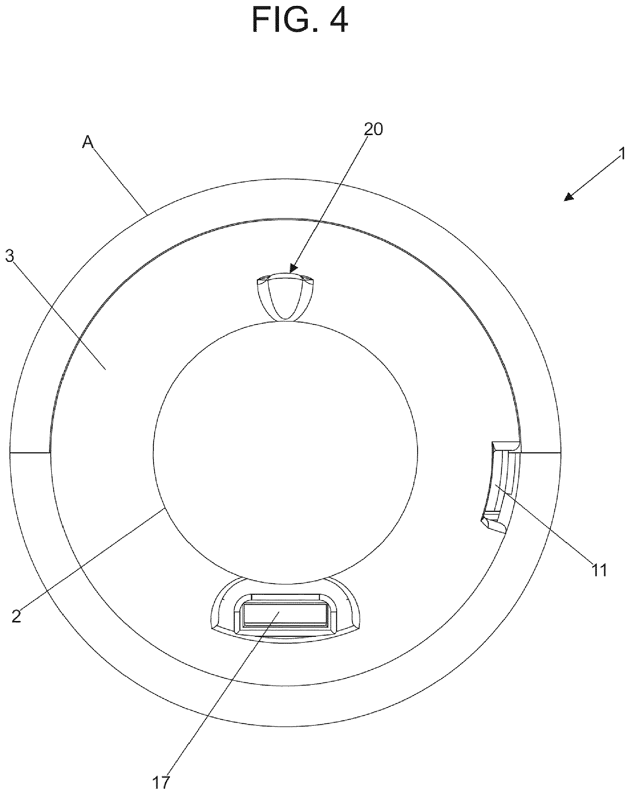

**FIGURE 4****:** Lower view of the fragrance-release devices;

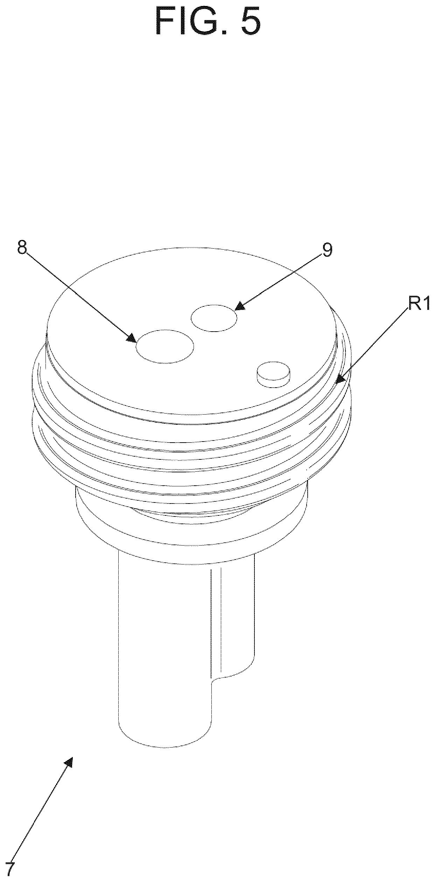

**FIGURE 5****:** Perspective view of the pump suction tube of the fragrance-release devices;

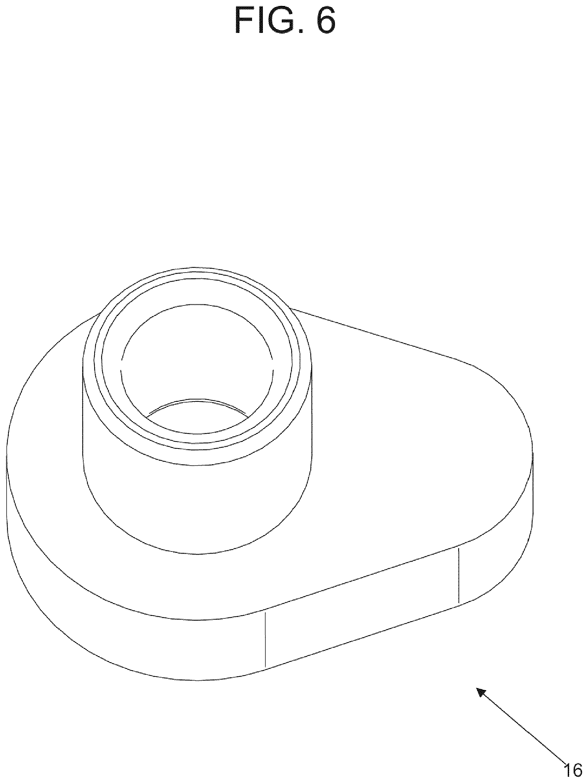

**FIGURE 6****:** Perspective view of the connector of the fragrance-release devices;

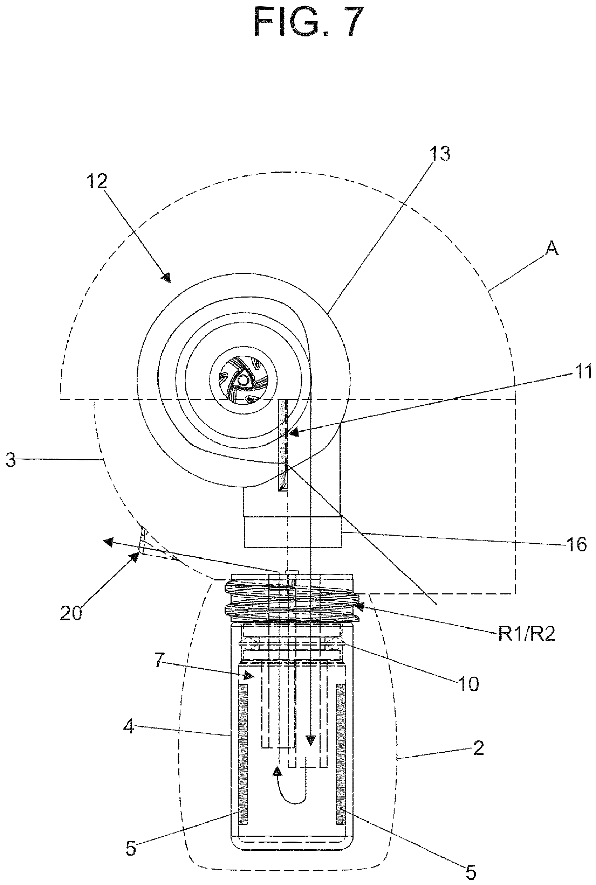

**FIGURE 7****:** Cross-section side view of the fragrance-release devices, showing the air flow during use with more fluid essence;

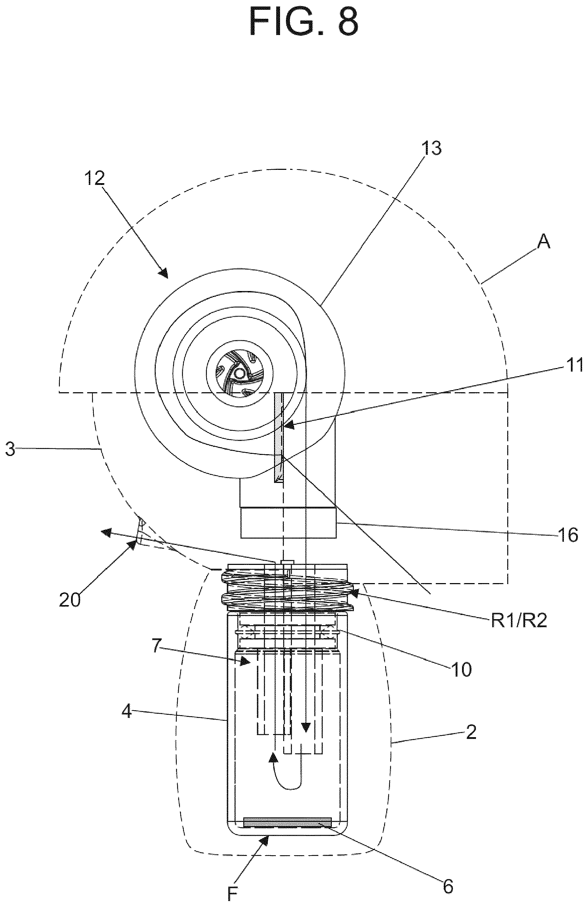

**FIGURE 8****:** Cross-section side view of the fragrance-release devices, showing the air flow during use with denser essence;

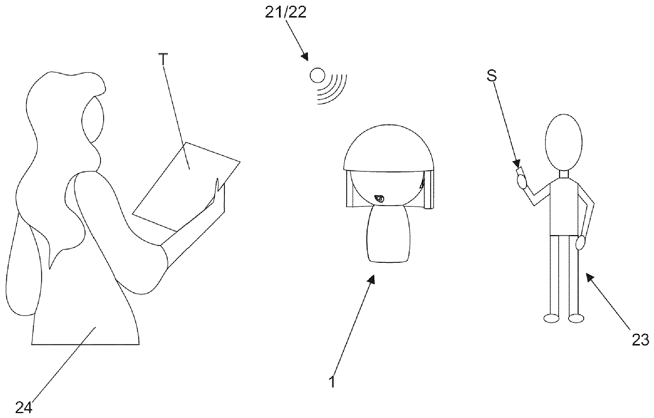

**FIGURE 9****:** Architecture of the use of the fragrance-release device.

### DETAILED DESCRIPTION OF THE INVENTION

**"FRAGRANCE-RELEASE DEVICE",** refers to a device formed by a base (2) containing the refill (5 or 6) and a couplable lid (3) wherein an air pump (12) actuated locally or remotely via message app, forces the air from the housing (13) to an ambient-air intake duct (8) pressurizing the container (4) with the refill (5 or 6), which naturally directs it to the outlet duct (9) of fragranced-air, releasing, in the form of a dry air spray, through the channel (20) for diffusion of fragranced-air, located on the lid (3) of the device (1).

More particularly, the invention refers to a device (1) for releasing fragrance in ornament, in this embodiment of the invention a miniature doll, which is constituted by a base (2) and a lid (3) with upper cover (A) which are adaptable to each other by means of a thread (R1 and R2) or other known means. The base (2) comprises a capsule with sufficient lower area to maintain the device (1) stable on a flat surface and the interior with volume to accommodate a cylindrical container (4) wherein the refill is placed with the fluid essence (5) or the dense essence (6), which is fixed in a non-definitive manner to a pump suction tube (7) with an ambient-air intake duct (8) and an fragranced-air outlet duct (9), whereby the quality of the aroma is guaranteed by means of a sealing ring (10), positioned on the neck of said pump suction tube (7), which avoids the entry of oxygen in the essence contained in the container (4). The pump suction tube (7) is fixed in a definitive manner to the lower end of the second part of the device (1), that is, to the lid (3), so that in order to perform the change of refill (5 or 6) it suffices to unscrew the base (2) and remove the container (4) to have access to the absorbent refill for fluid essence (5) or to the bottom (F) of the container (4) to replace the refill with a denser essence (6). In its turn, the lid (3) presents an external air intake duct (11) toward the air pump (12) formed by a housing (13), an electric motor (14), a turbine rotor (15), a connector (16), a power input (17) for battery (19) charging source (18) and a channel (20) for diffusion of fragranced-air. When the device (1) is energized and has its battery (19) charged it goes into operation automatically. Connected locally or remotely via message app, the electric motor (14) is driven by pulling the air through the external air intake conductor (11) which, when entering the housing (13) is forced toward the connector (16) to pass through the concentric orifice thereof to the ambient-air intake duct (8) which is longer than the fragranced-air outlet duct (9), being both located on the pump suction tube (7) which, pressurized, induces the fragranced dry air spray to the fragranced-air diffusion channel (20) located on the lid (2) of the device (1) thus performing the aromatization of the ambience.

When the device (1) is energized and has its battery (19) charged it goes into operation automatically. Connected remotely through the data network (21) or internet (22) to the specific app service via tablet (T) or smartphone (S). After connecting the device (1) to the communication app the users (23 and 24) can send the aroma at a certain moment, adjusting the intensity and duration of the emission. After sending the trigger command by the emitter, and acceptance by the receiver, the electronic system is actuated causing the emission of scented air to the receiver of the message.

The circuit board (25) uses a radio frequency chip if it is within the short-range radio signal coverage of the cell phone, tablet or Wi-Fi.

## Claims

1. **FRAGRANCE-RELEASE DEVICE,** actuated locally or remotely via message application, wherein the device is constituted by a base (2) and a lid (3) with upper cover (A), adaptable to each other; the base (2) comprises a capsule with a container (4) where the refill is placed with the fluid essence (5) or the refill with the dense essence (6); the container (4) is fixed in a non-definitive manner to a pump suction tube (7) with an ambient-air intake duct (8) and an fragranced-air outlet duct (9), which also comprises a sealing ring (10); the lid (3) presents an external air intake conduct (11) toward the air pump (12) formed by a housing (13), an electric motor (14), a turbine rotor (15), a connector (16) , a power input (17) for battery (19) charging source (18) and a channel (20) for diffusion of fragranced-air; when the device (1) is energized and has its battery (19) charged it goes into operation automatically. Connected locally or remotely via message app, the electric motor (14) is driven by pulling the air through the external air intake conductor (11) which, when entering the housing (13) is forced toward the connector (16) to pass through the concentric orifice thereof to the ambient-air intake duct (8) which is longer than the fragranced-air outlet duct (9), being both located on the pump suction tube (7) which, pressurized, induces the fragranced dry air spray to the fragranced-air diffusion channel (20) located on the lid (2) of the device (1).

2. **FRAGRANCE-RELEASE DEVICE,** according to claim 1, wherein the pump suction tube (7) is definitively fixed to the lid (3).

3. **FRAGRANCE-RELEASE DEVICE,** according to claim 2, wherein, in order to perform the change of refill (5 or 6) it suffices to unscrew the base (2) and remove the container (4) to have access to the absorbent refill for fluid essence (5) or to the bottom (F) of the container (4) to replace the refill with a denser essence (6).
